# DEMANDE DE BREVET EUROPEEN

(11) **EP 1 623 737 A1**
(43) Date de publication de la demande: **08.02.2006**
(21) Numéro de dépôt: 05291652.5
(22) Date de dépôt: 03.08.2005
(51) Int. Cl.: A61N 1/368

(54) **Dispositif médical implantable actif comprenant un mode de resynchronisation des contractions ventriculaires pour le traitement de l'insuffisance cardiaque**

(30) Priorité: 04.08.2004 FR 0408609
(71) Demandeur: ELA MEDICAL, F-92541 Montrouge (FR)
(72) Inventeur: Ripart, Alain, 91190 Gif sur Yvette (FR)
(74) Mandataire: Dupuis-Latour, Dominique

(57) **Abrégé**

Ce dispositif comprend un générateur (30) relié à une électrode ventriculaire droite (12) et à une électrode ventriculaire gauche (22). Le générateur comprend deux bornes de sortie (14, 24) reliées aux électrodes ventriculaires (12, 22), et des moyens pour délivrer conjointement à ces électrodes des impulsions de stimulation respectives avec un délai interventriculaire ajustable (DIV). Selon l'invention, le générateur est un générateur de type stimulateur double chambre (40) comprenant une borne auriculaire (60), une borne ventriculaire (50), et des moyens pour délivrer conjointement aux bornes auriculaire et ventriculaire des impulsions de stimulation avec un délai atrio-ventriculaire ajustable (DAV). L'une (14) des bornes de sortie est la borne auriculaire (A) et l'autre (24) est la borne ventriculaire (V), le délai atrio-ventriculaire (DAV) étant ajusté à la valeur du délai interventriculaire (DIV).

## Description

L'invention concerne les « dispositifs médicaux implantables actifs » tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Ces « dispositifs » se composent d'un boîtier ou « générateur », comprenant une pile et l'électronique du dispositif, et de sondes connectées au générateur et pourvues d'électrodes de recueil/stimulation en contact avec des sites auriculaires ou ventriculaires du myocarde.

L'invention concerne plus particulièrement les dispositifs aptes à assurer une stimulation conjointe et permanente des ventricules gauche et droit afin de resynchroniser ces derniers.

En effet, en alternative ou en complément au traitement des troubles du rythme cardiaque, il a été proposé de traiter par stimulation biventriculaire certains troubles de contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* 25, n°3, pp. 170-176 qui fait le point des travaux sur ce sujet.

Le dispositif est alors indiqué pour traiter l'insuffisance cardiaque de manière à améliorer l'état hémodynamique général de ces patients, et cette thérapie a permis d'observer des résultats souvent spectaculaires pour des patients en insuffisance cardiaque en classe III non améliorée par les traitements classiques.

Les premiers dispositifs de ce type opéraient en stimulation synchrone, c'est-à-dire que les deux sites ventriculaires recevaient au même moment l'impulsion de dépolarisation (délai interventriculaire nul). Les dispositifs les plus récents, comme cela est décrit par exemple dans le EP-A-1 108 446 (ELA Medical) opèrent au contraire de manière synchrone et asservie, en appliquant entre les deux stimulations un délai interventriculaire (DIV) variable, ajusté de manière à resynchroniser la contraction des ventricules avec optimisation fine de l'état hémodynamique du patient.

Les dispositifs utilisés actuellement à cet effet sont les prothèses dites « multisite » dans lesquelles des électrodes sont placées en une pluralité de sites respectifs distincts comportant les deux sites ventriculaires, droit et gauche, et au moins un site auriculaire. Il peut s'agir d'une prothèse de type « triple chambre » (double stimulation ventriculaire et détection/stimulation auriculaire droite) ou « quadruple chambre » (double stimulation ventriculaire et double détection/stimulation auriculaire). Le EP-A-0 925 806 (ELA Medical) décrit un tel type de dispositif multisite, comportant notamment des moyens de sélection de la configuration de stimulation la plus appropriée en fonction du patient.

Le point de départ de l'invention est la constatation de ce que la plupart des patients recevant un tel dispositif multisite implanté sont des patients qui présentent une conduction auriculo-ventriculaire habituellement normale (où chaque évènement auriculaire est suivi d'une dépolarisation ventriculaire associée) et qui n'ont donc pas d'indication standard pour la pose d'un stimulateur implanté.

Les dispositifs multisite triple ou quadruple chambre utilisés à cet effet, lorsqu'ils sont convenablement paramétrés pour assurer une stimulation biventriculaire, donnent certes entièrement satisfaction sur le plan clinique.

Mais il s'agit de dispositifs relativement élaborés, donc coûteux et délicats à programmer, alors même que nombre de fonctions dont ils sont pourvus ne sont pas utilisées.

L'un des buts de l'invention est de proposer un dispositif médical implantable actif spécialement adapté à une stimulation conjointe des ventricules afin de resynchroniser ces derniers, avec une configuration plus simple que les dispositifs multisite triple ou quadruple chambre utilisés jusqu'à présent à cet effet.

Un autre but de l'invention est la réduction du coût unitaire des dispositifs destinés à la stimulation biventriculaire en reprenant pour cela une partie matérielle identique à ce qui existe déjà dans un stimulateur connu, autorisant par là même une mise en oeuvre de l'invention sans surcoût important, à partir d'un appareillage existant et éprouvé ne nécessitant qu'une simple adaptation du logiciel de pilotage.

L'idée de base de l'invention consiste à utiliser un générateur de stimulateur double chambre DDD, c'est-à-dire originellement destiné à une stimulation atrio-ventriculaire, et à adapter ce dispositif pour le rendre capable d'une stimulation biventriculaire en lieu et place d'une stimulation auriculo-ventriculaire.

Cette adaptation pourra être réalisée par une modification simple du logiciel de pilotage du générateur. Ainsi, la partie matérielle du dispositif étant identique à ce qui existe déjà pour un stimulateur DDD, la stimulation biventriculaire pourra être mise en oeuvre, grâce à l'invention, sans surcoût important.

De plus, cette adaptation pourra être effectuée sur un générateur double chambre DDD de conception relativement simple et robuste, rendant d'autant plus facile la programmation par le praticien au moment de l'implantation.

Ce générateur DDD adapté pour permettre une stimulation biventriculaire sera relié à des sondes de stimulation ventriculaire gauche et ventriculaire droite de type connu, et non modifiées, pour donner un dispositif complet susceptible d'assurer la stimulation conjointe et permanente des ventricules gauche et droit afin d'en resynchroniser la contraction et, par voie de conséquence, améliorer l'état hémodynamique général du patient.

Plus précisément, l'invention concerne un dispositif médical implantable comprenant un mode de resynchronisation des contractions ventriculaires pour le traitement de l'insuffisance cardiaque.

Un dispositif de ce type, par exemple divulgué par le EP-A-1 108 446 précité, comprend un générateur combiné à une première sonde, pourvue d'une électrode ventriculaire droite, et à une deuxième sonde, pourvue d'une électrode ventriculaire gauche. Le générateur comprend une première borne de sortie, reliée à l'électrode ventriculaire droite, et une deuxième borne de sortie, reliée à l'électrode ventriculaire gauche. Il comprend également des moyens de stimulation ventriculaire droite et gauche, aptes à délivrer conjointement aux électrodes ventriculaires gauche et droite des impulsions de stimulation respectives, ainsi que des moyens pour établir un délai interventriculaire ajustable entre les instants d'application des impulsions respectivement délivrées à l'électrode ventriculaire droite et à l'électrode ventriculaire gauche au cours d'un même cycle cardiaque.

De façon caractéristique de l'invention, le générateur est un générateur de type stimulateur double chambre, c'est-à-dire comprenant une borne auriculaire et une borne ventriculaire, des moyens de stimulation auriculaire et ventriculaire, aptes à délivrer conjointement aux bornes auriculaire et ventriculaire des impulsions de stimulation respectives, et des moyens pour établir un délai atrio-ventriculaire ajustable entre les instants d'application des impulsions respectivement délivrées à la borne auriculaire et à la borne ventriculaire. L'une desdites première et deuxième bornes de sortie du dispositif est la borne auriculaire du générateur double chambre, et l'autre borne de sortie en est la borne ventriculaire, le délai atrio-ventriculaire étant ajusté à la valeur dudit délai interventriculaire.

Les moyens pour établir un délai atrio-ventriculaire ajustable peuvent être soit des moyens aptes à donner une valeur positive, nulle ou négative à ce délai atrio-ventriculaire, soit des moyens aptes à donner une valeur positive ou nulle à ce délai atrio-ventriculaire, le dispositif comportant alors des moyens commutateurs programmables pour permuter les bornes auriculaire et ventriculaire.

Le dispositif peut avantageusement comprendre un capteur d'au moins un paramètre hémodynamique, combiné à des moyens d'asservissement aptes, en fonction d'un signal délivré par ce capteur, à faire varier le délai atrio-ventriculaire ajustable dans le sens de l'amélioration dudit paramètre hémodynamique.

En variante ou en complément, le dispositif peut comprendre des moyens d'analyse d'un paramètre représentatif du degré de désynchronisation entre ventricules sur un cycle cardiaque, combiné à des moyens d'asservissement aptes, en fonction d'un signal délivré par ces moyens d'analyse, à faire varier le délai atrio-ventriculaire ajustable dans le sens de la diminution de ce degré de désynchronisation. On va maintenant décrire un exemple de mise en oeuvre du dispositif de stimulation biventriculaire de l'invention, en référence au dessin annexé où la figure unique illustre schématiquement un dispositif selon l'invention, et la manière de le réaliser à partir d'un générateur double chambre DDD.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody.*

Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

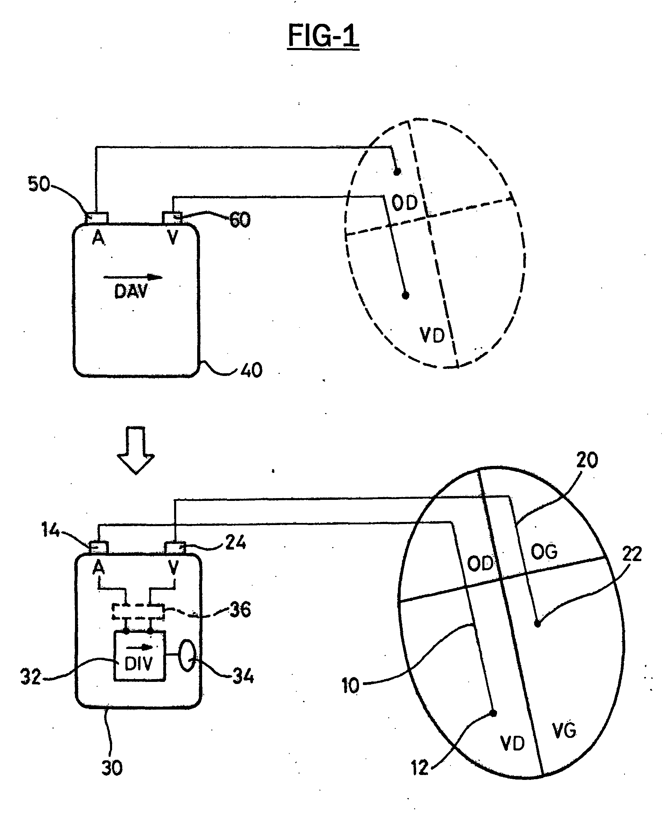

La figure 1 représente très schématiquement un muscle cardiaque, avec ses quatre cavités : oreillette droite OD, oreillette gauche OG, ventricule droit VD et ventricule gauche VG.

Pour permettre une stimulation biventriculaire, des sondes ont été implantées pour la stimulation de chacun des ventricules. Le ventricule droit VD reçoit une sonde 10 comportant une électrode ventriculaire droite 12. Il s'agit d'une sonde de type connu, par exemple celle présentée dans le EP-A-0 950 426 (ELA Medical), qui ne sera donc pas décrite plus en détail.

Le ventricule gauche VG, quant à lui, est stimulé par une sonde 20 pourvue d'une électrode ventriculaire gauche 22. La cavité ventriculaire gauche n'étant pas directement accessible, sa stimulation est opérée par une sonde enfilée dans le sinus coronaire (comme celle décrite dans le EP-A-1 374 945 (ELA Medical)) ou par une sonde épicardique, dont l'électrode 22 vient se placer sur la paroi externe du myocarde en un site permettant la stimulation du ventricule gauche.

Les électrodes 12 et 22 des sondes 10 et 20 sont reliées à des bornes respectives 14 et 24 d'un générateur 30 comprenant divers circuits d'analyse des signaux, de stimulation et de commande pilotés par un logiciel approprié, ces circuits étant schématisés par le bloc fonctionnel 32.

Pour permettre une resynchronisation satisfaisante de la contraction des ventricules avec optimisation fine de l'état hémodynamique du patient, un délai interventriculaire DIV est appliqué entre les instants respectifs de stimulation des deux ventricules gauche et droit.

En effet, une stimulation simultanée des deux ventricules n'est pas nécessairement optimale, car elle n'aboutit pas forcément à une contraction synchrone des deux ventricules, car les délais de conduction au sein du myocarde ne sont pas les mêmes à droite et à gauche et peuvent dépendre de multiples facteurs, ainsi que de l'emplacement de la sonde ventriculaire gauche, selon que celle-ci est une sonde enfilée dans le sinus coronaire ou une sonde épicardique.

Il est donc avantageux d'établir un délai interventriculaire entre les deux stimulations, et d'ajuster ce délai de manière à resynchroniser la contraction des ventricules et aboutir ainsi à une optimisation fine de l'hémodynamique.

L'introduction d'un délai interventriculaire de stimulation et son ajustement peuvent être réalisés par une programmation appropriée du microprocesseur du dispositif de manière à déclencher les différentes stimulations aux instants appropriés, ou par de la logique câblée, ou par une combinaison de moyens matériels et logiciels.

Le délai interventriculaire pourra être :
- nul,
- positif, le ventricule gauche étant stimulé après le ventricule droit avec un délai pouvant atteindre par exemple 48 ms, ou
- négatif, le ventricule droit étant stimulé après le ventricule gauche avec un délai pouvant atteindre par exemple 48 ms.

Ce délai interventriculaire est asservi à un signal directement ou indirectement représentatif du degré de synchronisation des contractions ventriculaires.

Le délai interventriculaire est ajusté à intervalles périodiques, en fonction de paramètres hémodynamiques mesurés en externe (par exemple par échographie) ou en continu par un capteur implanté 34 (capteur d'accélération endocavitaire, capteur de bioimpédance intracardiaque, etc.) délivrant au microprocesseur un indicateur du degré de synchronisation des contractions des ventricules droit et gauche.

Ces techniques d'analyse de l'état hémodynamique du patient et d'ajustement des paramètres de fonctionnement d'un dispositif asservi sont en elles-mêmes connues et ne seront pas décrites plus en détail. On pourra se référer notamment, outre le EP-A-1 108 446 précité, au EP-A-1 116 497 (ELA Medical) qui expose la manière de recueillir un signal de bioimpédance intracardiaque pour faire varier le délai interventriculaire d'application des impulsions respectives de stimulation des ventricules droit et gauche dans le sens de l'amélioration du débit cardiaque, et donc de la fraction d'éjection qui est le paramètre hémodynamique de référence.

De façon caractéristique de l'invention, le générateur 30 utilisé pour cette stimulation biventriculaire à délai interventriculaire variable est un générateur de stimulateur double chambre originellement prévu pour une stimulation atrio-ventriculaire (générateur DDD).

Un tel générateur 40 comprend, entre autres, une borne auriculaire 50, destinée à être reliée à une sonde pourvue d'une électrode implantée dans l'oreillette droite OD, et une borne ventriculaire 60, destinée à être reliée à une sonde pourvue d'une électrode implantée dans le ventricule droit VD. Un tel générateur doit en outre pouvoir appliquer entre les instants de stimulation de l'oreillette et du ventricule un délai atrio-ventriculaire DAV ajustable.

Les EP-A-1 048 322 et EP-A-1 050 320 (ELA Medical) décrivent de tels générateurs DDD comprenant des moyens pour reprogrammer si nécessaire le délai atrio-ventriculaire, de façon automatique.

Pour opérer une stimulation biventriculaire en lieu et place d'une stimulation atrio-ventriculaire, il suffit de connecter à ce générateur DDD 40 une sonde de stimulation ventriculaire droite 10 et une sonde de stimulation ventriculaire gauche 20 respectivement aux bornes 50 et 60, et de modifier le logiciel de pilotage du délai atrio-ventriculaire DAV de manière à ajuster ce délai à la valeur voulue du délai interventriculaire DIV.

Si le générateur DDD 40 permet, techniquement, de programmer le DAV à une valeur indifféremment négative, nulle ou positive (bien que, physiologiquement, la stimulation de l'oreillette doive toujours précéder celle du ventricule), le circuit du générateur d'impulsion pourra être directement utilisé pour la stimulation biventriculaire, en le pilotant de la manière indiquée plus haut par l'information représentative de l'état hémodynamique du patient.

Si le générateur 40 ne permet pas de programmer de délai atrio-ventriculaire négatif, il sera alors nécessaire d'ajouter au générateur 30 de stimulation biventriculaire des moyens commutateurs programmables 36 aptes à permuter électriquement les deux bornes 14 et 24 pour pouvoir stimuler un ventricule indifféremment en avance ou en retard sur l'autre, selon la position de ce commutateur 36.

## Revendications

1. Un dispositif médical implantable actif tel que stimulateur cardiaque, défibrillateur ou cardioverteur, comprenant un mode de resynchronisation des contractions ventriculaires pour le traitement de l'insuffisance cardiaque, ce dispositif comprenant :
- une première sonde (10), comportant une électrode ventriculaire droite (12),
- une deuxième sonde (20), comportant une électrode ventriculaire gauche (22), et
- un générateur (30), comprenant :
· une première borne de sortie (14), reliée à l'électrode ventriculaire droite (12), et une deuxième borne de sortie (24), reliée à l'électrode ventriculaire gauche (22),
· des moyens de stimulation ventriculaire droite et gauche, aptes à délivrer conjointement aux électrodes ventriculaires gauche et droite des impulsions de stimulation respectives, et
· des moyens pour établir un délai interventriculaire ajustable (DIV) entre les instants d'application des impulsions respectivement délivrées à l'électrode ventriculaire droite et à l'électrode ventriculaire gauche au cours d'un même cycle cardiaque,
dispositif **caractérisé en ce que** :
- ledit générateur est un générateur de type stimulateur double chambre (40) comprenant :
· une borne auriculaire (60) et une borne ventriculaire (50),
· des moyens de stimulation auriculaire et ventriculaire, aptes à délivrer conjointement aux bornes auriculaire et ventriculaire des impulsions de stimulation respectives, et
· des moyens (32) pour établir un délai atrio-ventriculaire ajustable (DAV) entre les instants d'application des impulsions respectivement délivrées à la borne auriculaire et à la borne ventriculaire,
- l'une (14) desdites première et deuxième bornes de sortie est ladite borne auriculaire (A) et l'autre (24) desdites première et deuxième bornes de sortie est ladite borne ventriculaire (V), et
- ledit délai atrio-ventriculaire (DAV) est ajusté à la valeur dudit délai interventriculaire (DIV).

2. Le dispositif de la revendication 1, dans lequel lesdits moyens (32) pour établir un délai atrio-ventriculaire ajustable (DAV) sont des moyens aptes à donner une valeur positive, nulle ou négative à ce délai atrio-ventriculaire.

3. Le dispositif de la revendication 1, dans lequel lesdits moyens (32) pour établir un délai atrio-ventriculaire ajustable (DAV) sont des moyens aptes à donner une valeur positive ou nulle à ce délai atrio-ventriculaire, et le dispositif comporte en outre des moyens commutateurs programmables (36) pour permuter les bornes auriculaire (A) et ventriculaire (V).

4. Le dispositif de la revendication 1, comprenant en outre :
- un capteur (34) d'au moins un paramètre hémodynamique, et
- des moyens d'asservissement aptes, en fonction d'un signal délivré par ce capteur, à faire varier ledit délai atrio-ventriculaire ajustable dans le sens de l'amélioration dudit paramètre hémodynamique.

5. Le dispositif de la revendication 1, comprenant en outre :
- des moyens d'analyse d'un paramètre représentatif du degré de désynchronisation entre ventricules sur un cycle cardiaque, et
- des moyens d'asservissement aptes, en fonction d'un signal délivré par ces moyens d'analyse, à faire varier ledit délai atrio-ventriculaire ajustable dans le sens de la diminution dudit degré de désynchronisation.
